(19) Europäisches Patentamt
European Patent Office
Office européen des brevets

(11) **EP 1 797 956 B1**

(12) **EUROPEAN PATENT SPECIFICATION**

(45) Date of publication and mention
of the grant of the patent:
**10.03.2010 Bulletin 2010/10**

(51) Int Cl.:
*B01L 3/00* (2006.01)    *B01J 19/00* (2006.01)
*C12M 1/42* (2006.01)    *C12Q 1/68* (2006.01)
*G01N 1/40* (2006.01)

(21) Application number: **06126171.5**

(22) Date of filing: **14.12.2006**

(54) **System and method for concentration and lysis of cells or viruses**

System und Methode zur Konzentrierung und Lyse von Zellen oder Viren

Système et procédé pour la concentration et pour la lyse des cellules ou viruses

(84) Designated Contracting States:
**DE FR GB**

(30) Priority: **14.12.2005   KR 20050123161**

(43) Date of publication of application:
**20.06.2007   Bulletin 2007/25**

(73) Proprietor: **Samsung Electronics Co., Ltd.
Suwon-si, Gyeonggi-do (KR)**

(72) Inventors:
• **Cho, Yoon-kyoung
Samsung Adv. Inst. Technologie
Gyeonggi-do (KR)**
• **Lee, Jeong-gun
Samsung Adv. Inst. Technologie
Gyeonggi-do (KR)**
• **Jeong, Sung-young
Samsung Adv. Inst. Technologie
Gyeonggi-do (KR)**

(74) Representative: **Grünecker, Kinkeldey,
Stockmair & Schwanhäusser
Anwaltssozietät
Leopoldstrasse 4
80802 München (DE)**

(56) References cited:
**WO-A-02/12896          US-A1- 2003 134 416
US-A1- 2004 018 611**

• **DHAWAN M.D., WISE F., BAEUMNER A.J.:
"Development of a laser-induced cell lysis
system" ANAL. BIOANAL. CHEM., [Online] vol.
374, no. 3, October 2002 (2002-10), pages 421-426,
XP002427260 Springer Berlin / Heidelberg ISSN:
1618-2650 Retrieved from the Internet: URL:http:
//www.springerlink.com/content/6d
hdrng87k8ly30x/fulltext.pdf> [retrieved on
2007-03-29]**

**Description**

BACKGROUND OF THE INVENTION

1. Field of the Invention

The present invention relates to a system for the concentration and the lysis of cells or viruses, more particularly to a system for concentrating and lysing cells or viruses in one chamber and a method of concentrating and lysing cells or viruses using the system.

2. Description of the Related Art

A biological analysis process including pathogen detection or molecular diagnosis is composed of target cell separation, cell concentration, biomolecule separation, biomolecule amplification, a hybridization reaction, and detection.

Research has been performed on a Lab-on-a-chip (LOC) in which a series of biologic analysis processes can be performed quickly and automatically on a microchip.

An LOC includes a microfluidic device to perform a biological analysis process. The microfluidic device includes an inlet, an outlet, a reaction chamber, and a microchannel connecting the inlet, the outlet, and the reaction chamber. The microfluidic device is equipped with a micropump for transferring a fluid, a micromixer for mixing the fluid, a microfilter for filtering the fluid, etc., in addition to the microchannel.

Examples of conventional devices for integrating a biologic analysis process can be found on the Internet at http://faculty.virginia.edu/landers/.

A conventional integrating system includes a cell counting chamber, a cell sorting chamber, a DNA extraction chamber, a PCR amplification chamber, and a detection chamber. The chambers are connected in sequence by a channel. Each of the chambers includes a valve and a pump.

However, when simply connecting various devices, which carry out the different processes of a biological analysis, it is difficult to integrate the various devices in one system. Due to the need for a plurality of valves and microfluidic controllers, such a system with various chambers connected in series has a large volume and is expensive.

Therefore, in order to reduce the size of an LOC, as many biological analysis processes as possible should be performed in one chamber.

US 2004/018611 and WO 02/12896 describe microfluidic systems.

SUMMARY OF THE INVENTION

The present invention provides a system for the concentration and the lysis of cells or viruses in one chamber according to claim 1.

The present invention also provides a method of concentrating and lysing cells or viruses using the system, according to claim 26.

By using the system and the method, cells or viruses can be concentrated and lysed effectively. Furthermore, the concentration and lysis can be performed in one chamber.

BRIEF DESCRIPTION OF THE DRAWINGS

The above and other features and advantages of the present invention will become more apparent by describing in detail exemplary embodiments thereof with reference to the attached drawings in which:

FIG. 1 is a schematic diagram of a microfluidic device used in an embodiment of the present invention;
FIGS. 2A and 2B are diagrams illustrating electrode structures used in an embodiment of the present invention;
FIGS. 2C and 2D are diagrams illustrating electrode structures used in another embodiment of the present invention;
FIG. 3A is a perspective exploded view of a reaction chamber used in an embodiment of the present invention;
FIG. 3B is a perspective view of a reaction chamber used in an embodiment of the present invention;
FIGS. 4 and 5A to 5D are diagrams illustrating the dimensions of an electrode portion used in an embodiment of the present invention;
FIG. 6 is a cross-sectional view illustrating the dimensions of a reaction chamber used in an embodiment of the present invention;
FIG. 7 is a graph showing the results of a polymerase chain reaction (PCR) of DNA extracted from E. coli cells subject to the magnetic bead density;
FIG. 8A is a diagram illustrating the trapping efficiency of various bacteria subject to the frequency of an applied voltage;

FIG. 8B is a diagram illustrating the trapping efficiency of magnetic beads subject to the frequency of an applied voltage;

FIG. 9 is a graph illustrating the concentration ratio subject to the flow rate of a fluid;

FIG. 10A is a photograph taken after flowing magnetic beads through a reaction chamber at a rate of 50 μl/min for one minute;

FIG. 10B is a photograph taken after flowing *Streptococcus mutans* at a rate of 250 μl/min for one second through the reaction chamber subsequent to flowing the magnetic beads through the reaction chamber;

FIG. 10C is a photograph taken after flowing *Streptococcus mutans* at a rate of 250 μl/min for 60 seconds through the reaction chamber subsequent to flowing the magnetic beads through the reaction chamber;

FIG. 11A is a photograph taken after flowing *Streptococcus mutans* at a rate of 250 μl/min for four minutes through a reaction chamber;

FIG. 11B is a photograph taken after flowing magnetic beads at a rate of 50 μl/min for one minute through the reaction chamber subsequent to flowing the *Streptococcus mutans* through the reaction chamber; and

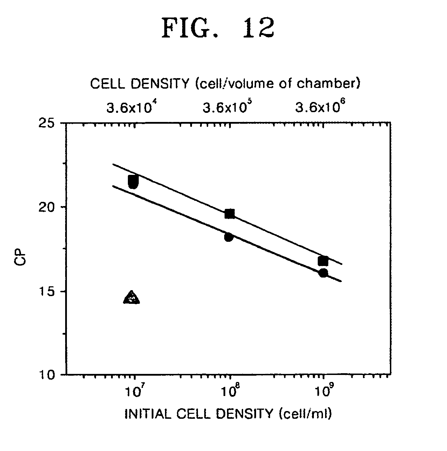

FIG. 12 is a graph showing the quantity of DNA determined in a sample solution that was applied to a chip by performing real - time PCR. The sample solution was obtained by: performing cytolysis by means of laser irradiation using a chip for cytolysis, without a previous concentration step; performing cytolysis by means of laser irradiation using a chip for cell concentration in which a metal electrode is patterned on glass, without a concentration step; and performing cytolysis by means of laser irradiation after a concentration step using a chip for cell concentration in which a metal electrode is patterned.

## DETAILED DESCRIPTION OF THE INVENTION

The present invention will now be described in detail with reference to the accompanying drawings, in which exemplary embodiments of the invention are shown. These embodiments are provided so that this disclosure will be thorough and complete, and will fully convey the concept of the invention to those skilled in the art. In the drawings, the thicknesses of layers and regions are exaggerated for the sake of clarity.

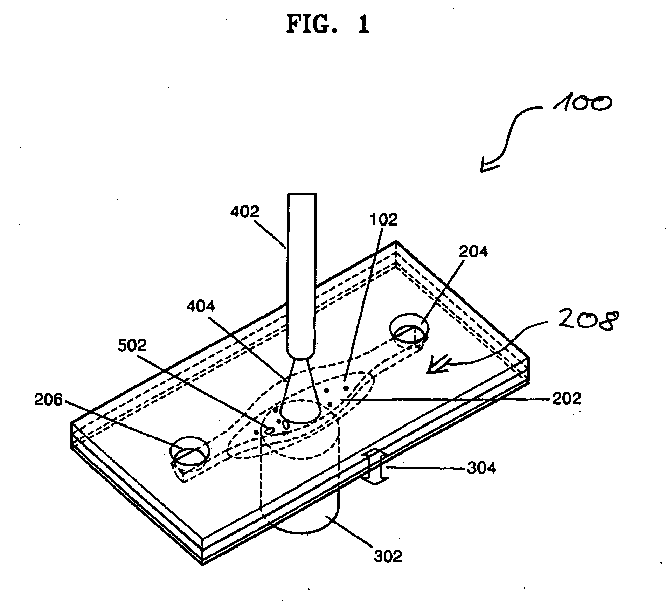

FIG. 1 is a schematic diagram of a microfluidic device 100 used in an embodiment of the present invention. The microfluidic device 100 can be used for the concentration and the lysis of cells or viruses 502.

Referring to FIG. 1, the microfluidic device 100 includes magnetic beads 102; a reaction chamber 202, in which the magnetic beads 102 are accommodated, and which reaction chamber 202 is equipped with electrodes 20, 20' separated by a dielectric (not shown) for generating a non-uniform electric field; a vibrating part 302 for agitating the magnetic beads 102 in the reaction chamber 202; and a laser generator 402 for cell lysis by irradiating a laser beam 404 to the cells 502 and magnetic beads 102 in the reaction chamber 202.

In an embodiment of the present invention, the size of the magnetic beads 102 may be 50 nm-1,000 μm, for example 1-50 μm. If the size of the particles is less than 50 nm, physical and mechanical impact power of the particles is not sufficient. Also, if the size of the magnetic beads is greater than 1,000 μm, the magnetic beads 102 are too large to be effectively used in a lab-on-a-chip (LOC). The magnetic beads 102 may be a mixture of magnetic beads having two or more sizes. In other words, the magnetic beads may include magnetic beads of one size or a mixture of magnetic beads of different sizes.

In an embodiment of the present invention, the magnetic beads 102 can be any beads that are magnetic. Particularly, they may have one or more materials selected from the group consisting of ferromagnetic metals such as Fe, Ni, and Cr and oxides thereof.

In another embodiment of the present invention, the magnetic beads 102 may include a polymer, an organic material, silicon, or glass coated with a ferromagnetic metal.

In an embodiment of the present invention, the surface of the magnetic beads 102 may have a negative charge, and thus DNA does not stick thereto. Since DNA has a negative charge, DNA does not stick to the surface due to a repulsive force if the surface also has a negative charge. If DNA sticks to the surface of the magnetic beads, it is difficult to refine DNA because then it is difficult to separate the magnetic beads and the DNA.

In an embodiment of the present invention, a functional group on the surface of the magnetic beads may be hydrophilic. The amplification efficiency of DNA obtained from the lysed cells may vary subject to the functional group on surface of the magnetic beads. The amplification efficiency of DNA increases with increasing hydrophilicity of the functional group on the magnetic beads' surface. The functional group may be negatively charged and have a carboxy radical or a derivative thereof. The derivative may be iminodiacetic acid (IDA), ethylene diamine tetraacetic acid (EDTA), citric acid, or polycarboxylic acid.

The reaction chamber 202 includes electrodes 20, 20' separated by a dielectric (not shown) for generating a non-uniform electric field. The reaction chamber 202 may have an inlet port 204 through which a fluid can be introduced into the reaction chamber 202 and an outlet port 206 through which the fluid can be removed out of the reaction chamber 202. The fluid may contain the magnetic beads 102, cells, or viruses 502. When pumping the fluid through the inlet port 204 into the reaction chamber 202, the fluid flows through the reaction chamber 202 in a flow direction 208, which is directed from the inlet port 204 towards the outlet port 206 in the embodiment shown in Fig. 1. Upon reaching the outlet port 206, the fluid is discharged from the reaction chamber 202.

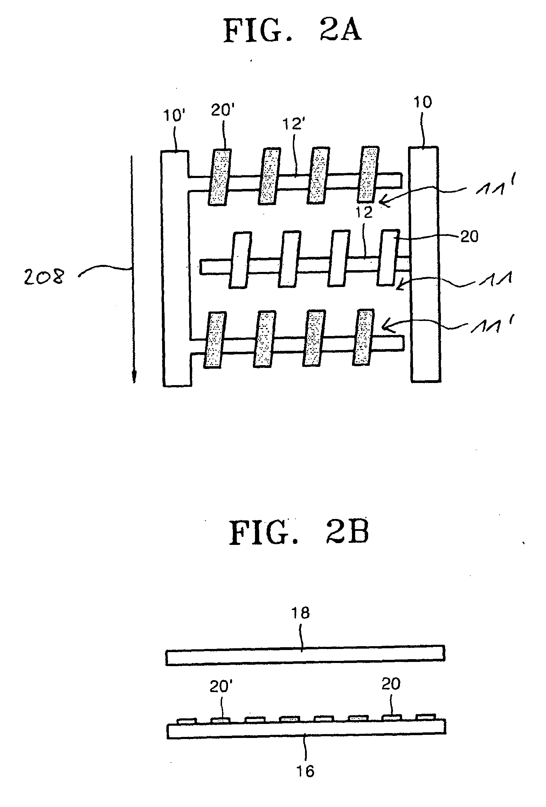

In an embodiment of the present invention, the electrodes 20, 20' for generating the non-uniform electric field are arranged staggered to each other. As shown in Figs. 2A to 2D for example, the electrodes are arranged in rows 11, 11', each row comprising two or more electrodes 20, 20', wherein said rows 11, 11' are aligned substantially perpendicularly to the direction 208 of fluid flow through the reaction chamber 202. Odd rows of electrodes 11' can be connected to a first metal pad 10' via metal lines 12' and even rows of electrodes 11 can be connected to second metal pad 10 via other metal lines 12.

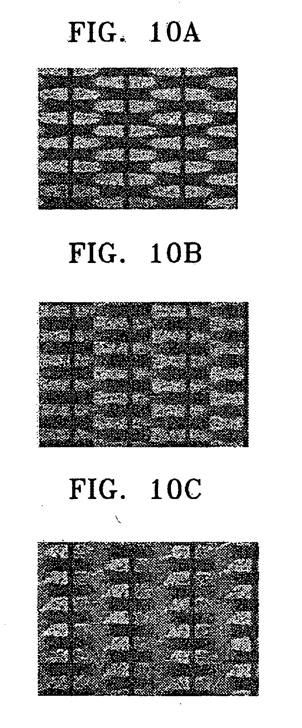

In an embodiment of the present invention, the electrodes 20 of an even row of electrodes 11 may extend into the neighboring odd row of electrodes 11', namely into the space between electrodes 20' of the adjacent odd rows of electrodes 11'. This embodiment is shown for example in Figs. 10A to 10C.

In an embodiment of the present invention, the electrodes 20' of one odd row 11' are connected to the respective first metal pad 10' by means of one single metal line 12'. The electrodes 20 of one even row 11 may likewise be connected to the respective second metal pad 10 by means of one single metal line 12. The metal pad 10, 10' and the metal line 12, 12' can be composed of any metal. For example, the metal can be selected from the group of gold, copper, and platinum, and may be a biocompatible metal such as gold, which is desirable for cells or biomass.

In an embodiment of the present invention, the distance D between an electrode 20' of an odd row 11' and an electrode 20 of an even row 11 can be 10-100 $\mu$m. The distance P between adjacent electrodes 20' within an odd row 11' and between adjacent electrodes 20 within an even row 11 can be 10-100 $\mu$m. The thickness of the electrodes 20, 20' can be 0.1-100 $\mu$m, for example, 50-100 $\mu$m.

In an embodiment of the present invention, the electrodes 20, 20' can be columnar, for example of cylindrical or square pillar shape. Alternatively, the electrodes 20, 20' can be flat type electrodes. However, the shape of the electrodes 20, 20' that can be used in embodiments of the present invention is not limited to the above examples, and the electrodes 20, 20' can have any form.

In an embodiment of the present invention, a surface of the electrodes can be arranged at an angle from 50 to 120° to the surface of the substrate. If the electrodes have a square pillar form, they can have an overturned three-dimensional trapezoid shape, and if the electrodes have a cylindrical form, they can have an overturned conical shape with a flat end.

In an embodiment of the present invention, the electrodes 20, 20' can be gold coated on a metal. The metal may be nickel, for example.

In an embodiment of the present invention, the chamber 202 can be composed of any material, such as glass, silicon, Pyrex, quartz, or SU-8. The material forming the chamber 202 may be a transparent material such as glass, Pyrex, quartz, or SU - 8.

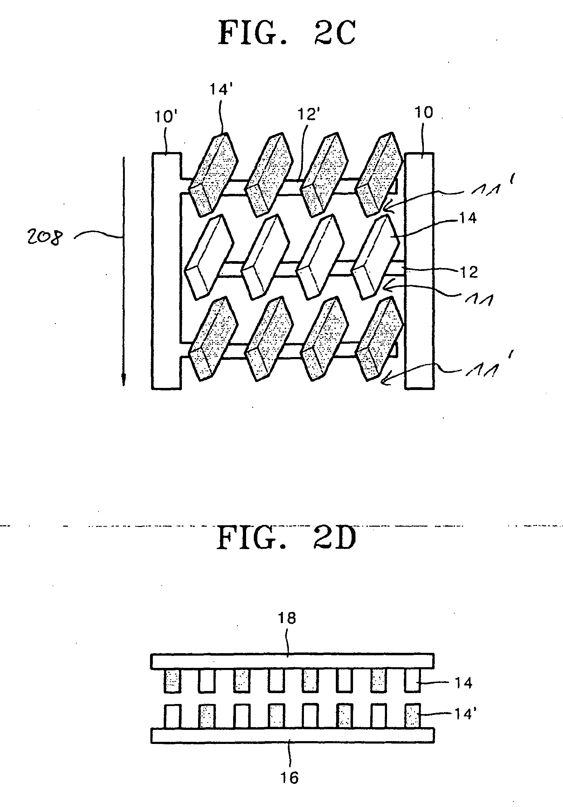

In an embodiment of the present invention, the chamber has a lower substrate 16 and an upper substrate 18. The electrodes 20, 20' can be disposed on the lower substrate 16 and/or the upper substrate 18. A chamber 202 including a lower substrate 16 and an upper substrate 18 can be manufactured by making a lower substrate 16 and an upper substrate 18 separately and then joining them, but the present invention is not limited to this example. To join the lower substrate 16 and the upper substrate 18, any bonding method, which is known to those of ordinary skill in the art, can be used. For example, an adhesive tape 22, which is commercially purchasable from 3M, can be used. In an embodiment of the present invention, rows of the electrodes 11, 11' disposed on the lower substrate 16 and rows of the electrodes 11, 11' disposed on the upper substrate 18 correspond to each other, this means that said rows of electrodes 11, 11' are arranged spaced apart from but substantially congruent with each other in the direction perpendicular to the plane of the substrate 16 and 18. The electrodes 20' of the odd rows 11' on the lower substrate 16 and the electrodes 20 of the even rows 11 on the upper substrate 18 are connected to the same metal pad 10 or 10'. The electrodes 20 of the even rows 11 on the lower substrate 16 and the electrodes 20' of the odd rows 11' on the upper substrate 18 can all be connected to the same, other metal pad 10' or 10, respectively. This embodiment is shown for example in Figs. 2C and 2D.

FIGS. 2A and 2B are diagrams illustrating electrode structures according to an embodiment of the present invention. FIGS. 2C and 2D are diagrams illustrating electrode structures according to another embodiment of the present invention.

Referring to FIG. 2A, rows 11, 11' of metal plates 20 and 20', which metal plates 20, 20' serve as the electrodes in the embodiment of Fig. 2A, are disposed perpendicularly to the flow direction 208 of the fluid. Each of the rows 11, 11' includes four metal plates 20 or 20'. Moreover, the metal plates 20' of one odd row 11' are connected to a first metal pad 10' through a metal line 12'. The metal plates 20 of one even row 11 are connected to another second metal pad 10 through a metal line 12. As shown in FIG. 2B, the device includes an upper substrate 18 and a lower substrate 16. The metal plates 20 and 20' can be disposed on the surface of the lower substrate 16, the upper substrate 18, or on both the lower substrate 16 and the upper substrate 18.

Referring to FIG. 2C, rows 11, 11' of metal posts 14 and 14', which metal posts 14, 14' serve as the electrodes in the embodiment of Fig. 2C, are disposed perpendicularly to the flow direction 208 of the fluid. Each of the rows 11, 11' includes a plurality of metal posts 14 or 14'. Moreover, the metal posts 14' of odd rows 11' are connected to a first metal pad 10' through a metal line 12', and the metal posts 14 of even rows 11 are connected to another second meal pad 10' through a metal line 12. As shown in FIG. 2D, the device includes an upper substrate 18 and a lower substrate 16. The metal posts 14 are disposed on the lower substrate 16 and the upper substrate 18.

In an embodiment of the present invention, the vibrating part 302 may include an ultrasonic cleaner, a vibrating unit that operates in response to a magnetic field, a vibrating unit that operates in response to an electric field, a mechanical vibrating unit such as a vortex, etc., or piezoelectric materials. The vibrating part 302 can be attached to the reaction chamber 202, and can be any device that can vibrate a mixture of cells 502 and micro magnetic beads 102 accommodated in the reaction chamber 202.

The vibration direction of the vibrating part 302 can be any direction, for example, a vertical direction or a horizontal direction. Referring to FIG. 1, the vibrating part 302 vibrates the reaction chamber 202 including the magnetic beads 102 in a vertical direction 304.

In an embodiment of the present invention, the laser 402 can be a pulse laser or a continuous wave laser.

If the output power of the laser 402 is too low, a laser ablation phenomenon cannot occur efficiently. The output power can be more than 10 mW for a continuous wave laser and more than 1 mJ/pulse for a pulse laser. The output power of the pulse laser may be 3 mJ/pulse or greater, and the output power of the continuous wave laser may be 100 mW or greater. If the output power of the continuous wave laser is less than 10 mW or the output power of the pulse laser is less than 1 mJ / pulse, not enough energy is transmitted to destroy the cells.

In an embodiment of the present invention, the laser 402 generates a laser beam 404 having a specific wavelength band that can be absorbed by the magnetic beads 102. The laser 402 may generate light 404 in the wavelength band of 400 nm or greater, for example, in the wavelength band of 750-1300 nm, because the problem of denaturizing or damaging of DNA occurs with a wavelength of less than 400 nm. Moreover, the laser 402 can generate one or more wavelength bands. That is, the laser 402 can have one wavelength in the above wavelength range or two or more different wavelengths.

A method of concentrating or lysing cells 502 or viruses using the microfluidic device 100 and laser beam 404 according to an embodiment of the present invention will now be described.

When performing sequentially the step of concentrating cells 502 in the reaction chamber 202 by means of dielectrophoresis (DEP) using the electrodes generating a non-uniform electric field and the step of cytolysing the cells 502 by means of a laser 402 and magnetic beads 102, several problems can arise.

For example, if a magnetic bead 102 containing solution is injected into a reaction chamber 202 after concentrating cells 502 in the reaction chamber 202, the following problems are likely to occur: the dilution of the concentrated sample; difficulty of control of a small chamber volume; and the generation of bubbles during the injection of an additional solution.

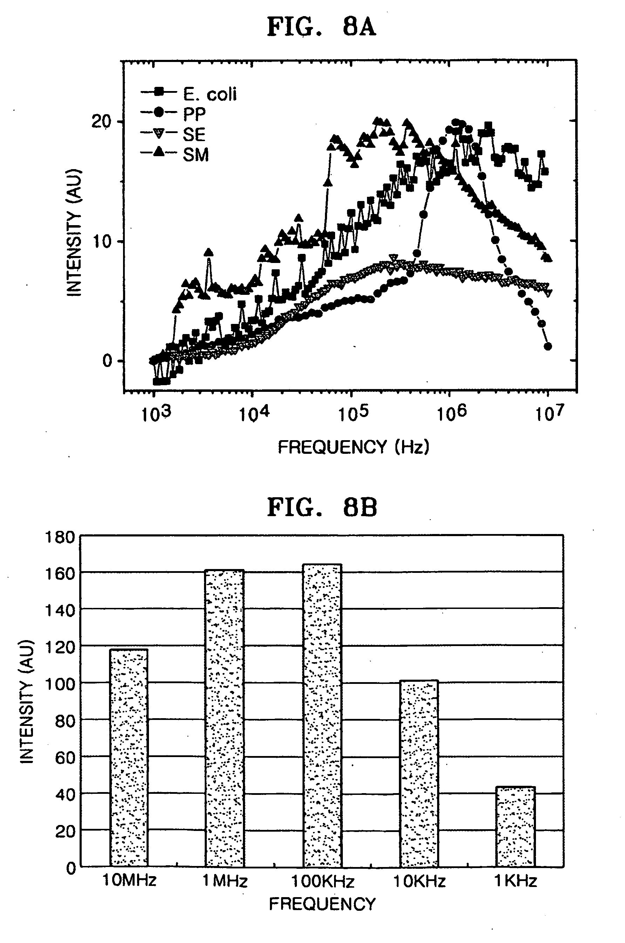

It has been confirmed by the inventors of the present invention that bacteria 502 and the magnetic beads 102 used for laser lysis behave similar during DEP and that the magnetic beads 102 and the bacteria 502 were both captured in the non-uniform electric field when applying a similar voltage frequency, as shown in FIGS. 8A and 8B. The preferred conditions for the concentration and lysis method of cells 502 or viruses were obtained using this information.

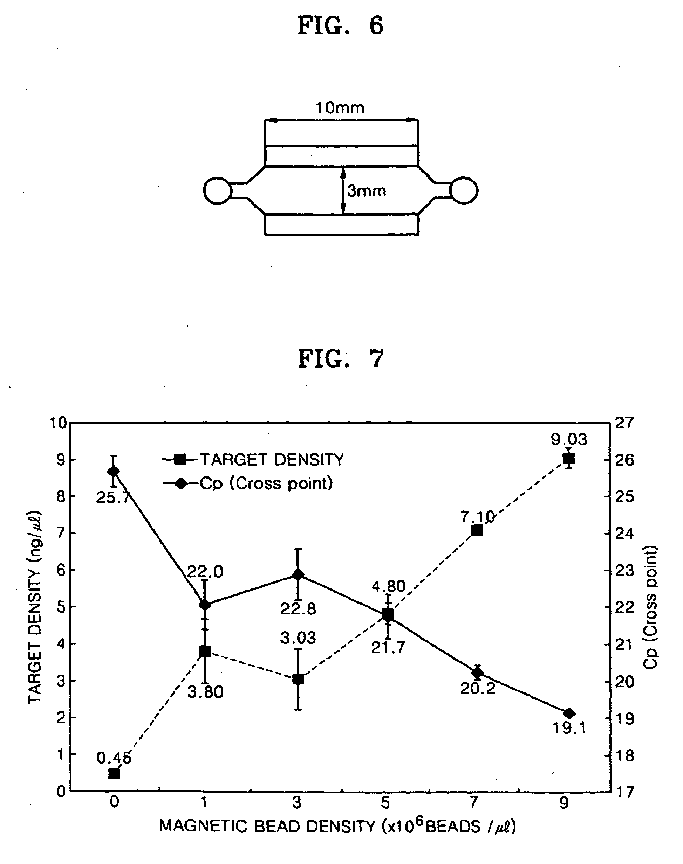

First, when a magnetic bead 102 containing solution and cells 502 were mixed in the reaction chamber 202, the loss of the magnetic bead 102 was significant (not illustrated.) This is not desirable since a higher magnetic bead density ensures better cytolysis efficiency, as illustrated in FIG. 7.

Moreover, if cells 502 are concentrated in the reaction chamber 202 after capturing magnetic bead 102, the already captured magnetic beads 102 are washed out of the reaction chamber 202 if a sample solution for cell concentration is flowed at a high flow rate through the reaction chamber 202, as illustrated in FIGS. 10B and 10C. As the speed of the flow increases, cell concentration increases but cytolysis efficiency decreases because magnetic beads 102 that have been previously captured are removed, as illustrated in FIG. 7.

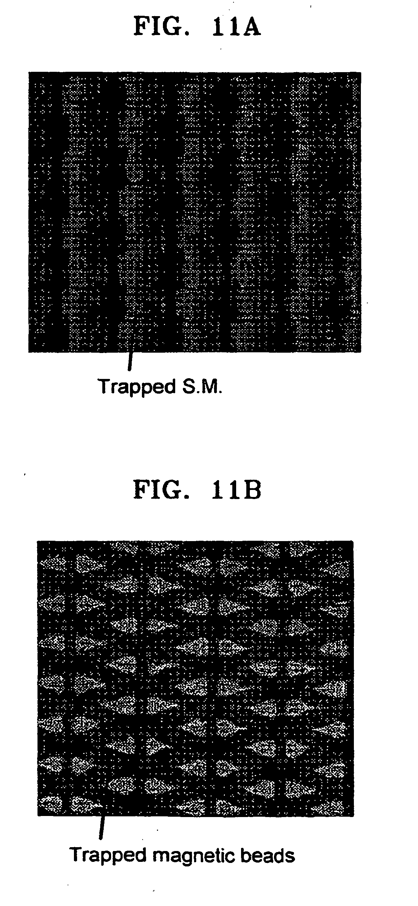

On the other hand, when removing a small amount of the magnetic bead 102 containing solution after capturing cells 502 in the reaction chamber, both the cells 502 and the magnetic beads 102 were captured very effectively in the reaction chamber when generating the non-uniform electric field by means of the electrodes 20, 20', as demonstrated in FIGS. 11A and 11B.

Therefore, a method of concentrating and lysing of cells 502 or viruses using the microfluidic device 100 and laser beam 404 according to an embodiment of the present invention may include: applying a voltage to the electrodes 20, 20' thereby generating a non-uniform electric field in the reaction chamber 202; flowing a fluid containing cells 502 or viruses into the reaction chamber 202; flowing a fluid containing magnetic beads 102 into the reaction chamber; and irradiating laser beam 404 onto the magnetic beads 102 present in the reaction chamber 202.

In an embodiment of the present invention, the applied voltage can have a magnitude of 1-100 V and a frequency of 100 Hz-100 MHz, preferably 100 kHz-100 MHz.

As described above, after generating a non-uniform electric field in the reaction chamber 202 and flowing the fluid containing the cells 502 or viruses into the reaction chamber 202, the cells 502 or viruses are captured and hence concentrated in the reaction chamber 202 of the microfluidic device 100 by DEP due to the non-uniform electric field generated according to an embodiment of the present invention.

Moreover, if the fluid having the magnetic beads 102 flows into the reaction chamber 202 after generating the non-uniform electric field in the chamber 202, the magnetic beads 102 are captured, in addition to the captured cells 502 or viruses, in the reaction chamber 202 of the microfluidic device by DEP according to an embodiment of the present invention.

In an embodiment of the present invention, the flow rate of the fluid flowing through the reaction chamber 202 can be 0.1 mm/sec or greater, for example, 1 mm/sec or greater.

When the laser beam 404 is irradiated onto the magnetic beads 102, the laser ablation phenomenon occurs. Thus, an impulse wave, vapor pressure, and heat are delivered to the cell surfaces, and the physical impacts thereof are applied simultaneously. The magnetic beads 102 heated by the laser beam 404 raise the temperature of the sample solution, and the hot magnetic beads 102 directly lyse cells 502 or viruses. However, the magnetic beads 102 in the sample solution do not simply act as heat transfer bodies, but also thermally, mechanically, and physically affect the cells' surfaces, thus effectively destroying the cell surfaces.

Denaturized proteins and cell debris adhere to the magnetic beads 102, which can be removed using a magnetic force or gravity. This allows a remarkable improvement in PCR analysis by lowering the detection limit, drastically reducing the time required for DNA extraction by reducing the DNA extraction process to one step only, and increasing the signal amplitude. It takes only 40 seconds to lyse cells 502 using the micro magnetic beads 102 and the laser 402.

In an embodiment of the present invention, the sample solution can be selected from the group consisting of spittle, urine, blood, blood serum and cell broth. The sample solution can include materials having nucleic acids such as animal cells, plant cells, bacteria, viruses, and phages. When the sample solution includes animal cells, plant cells, bacteria, or viruses, the conductivity of the sample solution can be less than 30 mS/m.

Using the method according to the present invention, one can obtain excellent results, which correspond to results that are obtained when performing cytolysis by means of laser irradiation without a previous concentration step, wherein 100 times or more DNA is used, as can be seen in Table 1 and FIG. 12.

The present invention will now be described in greater detail with reference to the following examples. The following examples are for illustrative purposes only and are not intended to limit the scope of the invention.

<Example 1>

Manufacture of microfluidic device used in the invention

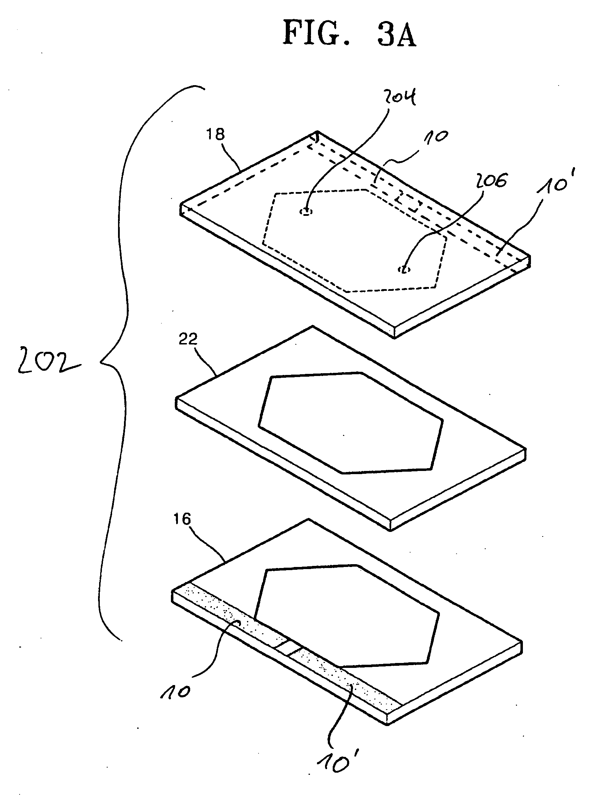

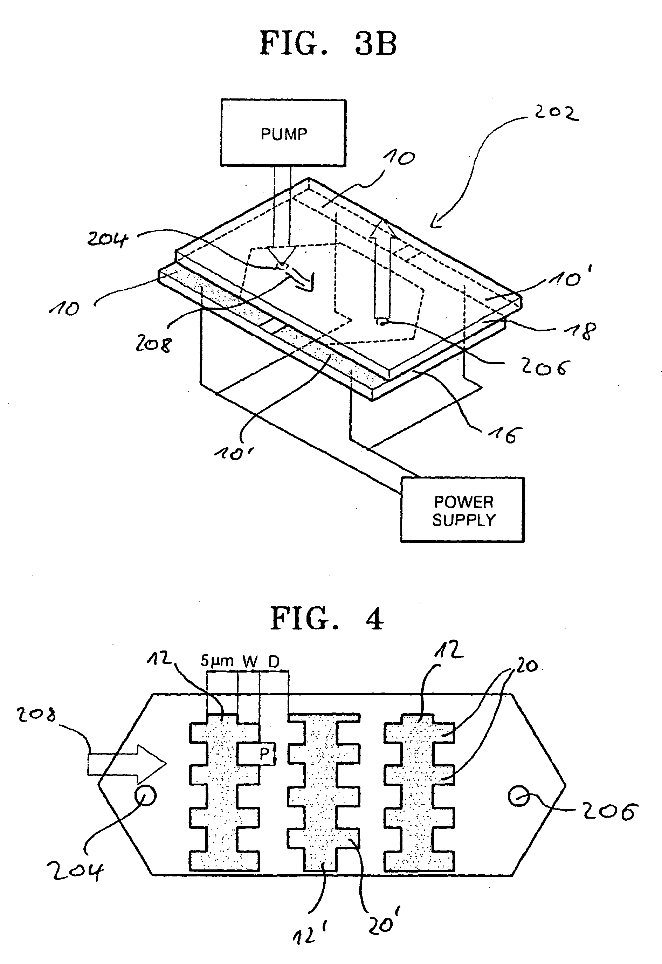

FIGS. 3A and 3B are respectively a perspective exploded view and a perspective view of a reaction chamber that was prepared. Referring to FIG. 3A, the reaction chamber 202 was formed by connecting an upper substrate 18 and a lower substrate 16 with 3 M adsorption tape 22 (3M, U.S.) On the lower substrate 16 an array of gold pillars constituting the electrodes 20, 20' was formed. FIG. 3B illustrates the manufactured reaction chamber. Electric power is provided by a power supply supplied to the electrodes via metal pads (10, 10'), which metal pads 10, 10' are connected via leads to the power supply. A fluid can be pumped through the reaction chamber 202 in a flow direction 208. The fluid enters the reaction chamber 202 via an inlet port 204 and leaves the chamber 202 via an outlet port 206, when the fluid is conveyed using a pump.

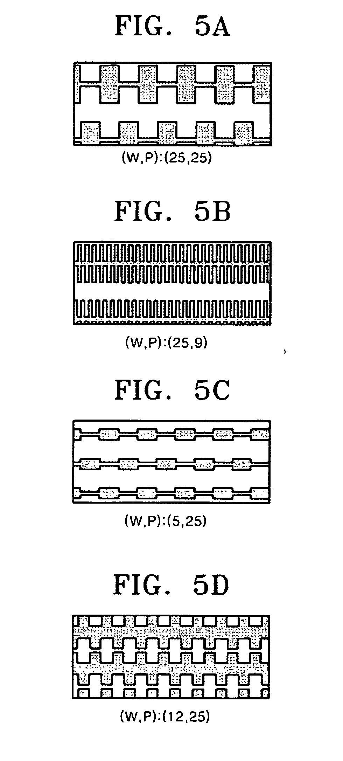

FIGS. 4 and 5 are diagrams illustrating the dimensions of the electrode means of Example 1 used to generate the non-uniform electric field in the chamber according to the embodiment of Figs. 3A and 3B.

Referring to FIG. 4, the width of a metal line 12, 12' connecting gold pillars 20, 20' to the metal pads 10, 10' was 5 $\mu$m. The distance between the adjacent gold pillars 20, 20' disposed on one metal line 12, 12' in the array of the gold posts was P. The width of the gold pillars 20, 20' extending beyond the metal line 12, 12' was W. The distance between the gold pillars from one row to the pillars of the next row was D. The arrow shows the flow direction of fluid 208 directed from the inlet port 204 to the outlet port 206.

All of the electrode structures illustrated in FIGS. 5A through 5D showed similar experiment results. Therefore, in the examples, experiments were performed with the microfluidic devices having the electrode dimension shown in FIG. 5A.

FIG. 6 is a cross-sectional view illustrating the dimensions of the reaction chamber 202. Referring to FIG. 6, the reaction chamber 202 had a length of 10 mm, a width of 3 mm, and a volume of 4 $\mu$l.

Micro magnetic beads 102 (Dynabeads MyOne™ Carboxylic Acid, DYNAL, Norway) were used as magnetic beads 102. A coin-shaped vibrating motor (DMJBRK 20 X, Samsung electro-mechanics, Republic of Korea) was used as a vibrating part 302. A high power laser, HLU 25 F 100-808 (LIMO, Germany), was used as a laser source 402.

<Example 2>

Cytolysis efficiency subject to magnetic bead density

Cytolysis efficiency subject to the magnetic bead density was investigated. Different quantities of beads were added to a sample solutions (0 to 9 x $10^6$ bead/$\mu$l) and the sample solutions containing *E. coli* cells 502 in addition to the magnetic beads 102 was irradiated with a 1 W laser beam having a wavelength of 808 nm for 40 seconds. FIG. 7 is a graph illustrating the polymerase chain reaction (PCR) results of DNA obtained from *E. coli* cells subject to magnetic bead density used. The crossing point (Cp) refers to the number of cycles in the real-time PCR reaction that are required to obtain a detectable fluorescence signal. The higher the initial DNA density, the lower the Cp. Moreover, the Cp is related to DNA refinement. The higher the DNA purity, the lower the Cp. Therefore, the Cp is lower when the DNA of the sample solution is more refined.

Referring to FIG. 7, a higher density of the magnetic beads 102 ensures that more DNA is obtained. High cytolysis efficiency was obtained and much DNA was extracted when the density of the magnetic beads 102 was 5 x $10^6$ beads/$\mu$l or more. Moreover, the Cp value of *E. coli* DNA amplification for the exact measurement of the initial target copy number was investigated using LightCycler (Roche Diagnostics Corporation, IN, USA), a publicly known real-time PCR instrument. Referring to FIG. 7, the Cp value reduces as the density of the magnetic beads increases. Thus, cytolysis efficiency increases as the density of magnetic beads increases.

<Example 3>

DEP trapping efficiency of bacteria and magnetic beads subject to frequency of voltage applied

Trapping efficiency subject to the frequency of the voltage applied was investigated by flowing each of *E. coli* (ATCC #11775), *Pseudomonas fluorescence* (ATCC #13525), and *Streptococcus mutans* (ATCC #35668), and the magnetic beads 102 of Example 1 through the reaction chamber 202.

Each species of these bacteria was conveyed in a solution having a density of $10^7$ cells/ml at a flow rate of 100 $\mu$l /min. The magnetic beads were transported at a density of $10^5$ beads/$\mu$l at a flow rate of 50 $\mu$l/min. The magnitude of the applied voltage was 20 V.

The results are shown in FIGS. 8A and 8B. FIG. 8A illustrates the trapping efficiency of each species of bacteria depending on the frequency of the voltage applied. FIG. 8B illustrates the trapping efficiency of the magnetic beads 102 subject to the frequency of the voltage applied. Referring to FIG. 8A and 8B, all of the bacteria 502 and the magnetic beads 102 showed an optimum trapping efficiency at a frequency of from 100 kHz to 1 MHz. Therefore, both the bacteria 502 and the magnetic beads 102 can be captured under the same DEP conditions.

<Example 4>

Cell trapping efficiency subject to flow rate of the fluid

The trapping efficiencies of *E. coli, Streptococcus mutans,* and *Staphylococcus epidermidis* were measured subject to the flow rate of the fluid.

Bacteria, which were not marked, were used. The trapping efficiency was calculated as the number of bacteria eluted, which was measured as the density (C output) of the solution that passed through a concentration chip to which an electric field was applied, divided by the number of bacteria injected, which was measured as the density of the initial bacteria suspension (C input) before application onto the concentration chip.

$$\text{Concentration ratio (times)} = \text{(density of eluted bacteria) / (density of input bacteria)}$$

Two different methods were used to measure the density of cells: a colony count method, which was used for a density of $10^6$ cells/ml or less, and the BacLight Bacterial viability kit (Molecular probes, U.S.), wherein the cells were marked with fluorescence, for a density of more than $10^6$ cells/ml. According to the manual of the kit used, fluorescent labeling was performed by adding 3 $\mu$l of a staining mixture of SYPO 9 and propidium iodide to 1 ml of a cell solution. After 20 minutes, the fluorescent intensity was measured using a SpectraMax Gemini XS 20. To determine the relative quantity, a standard curve was obtained by performing serial dilution of a bacterium solution (OD600=1), and the density of the cell solution was measured relative to this standard curve. The absolute quantity of the bacterium solution (OD600=1) was measured using a colony count method by diluting the solution.

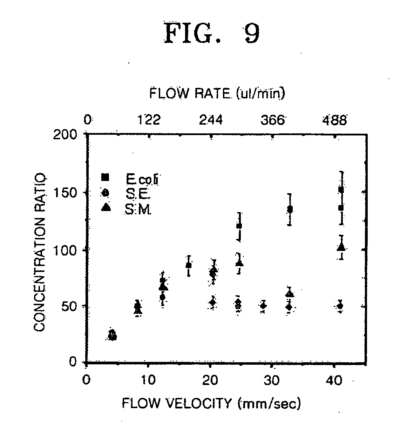

FIG. 9 is a graph illustrating the concentration ratio subject to the flow rate of the fluid. In FIG. 9, the concentration ratio is defined as (cell density of solution eluted with 10 μl buffer after 2 minutes of capturing)/(initial cell density). Referring to FIG. 9, the higher the flow velocity, the higher the concentration ratio, and a maximum concentration ratio is obtained at a specific flow velocity for each of the three bacteria tested.

<Example 5>

Trapping efficiency of magnetic beads and cells subject to the capture order

The trapping efficiency subject to the order in which the magnetic beads 102 and the cells 502 were captured was investigated using the conditions obtained in Example 3. The voltage applied had a magnitude of 20 V and a frequency of 100 kHz.

After a solution containing magnetic beads 102 having a density of $10^5$ beads/μl was transported through the reaction chamber 202 at a flow rate of 50 μl /min for one minute, a *Streptococcus mutans* solution having a density of $10^7$ cells/ml was subsequently conveyed through the reaction chamber 202 at a rate of 250 μl/min for one minute.

FIG. 10A is a photograph taken after flowing magnetic beads 102 through a reaction chamber 202 at a flow rate of 50 μl/min for one minute. FIG. 10B is a photograph taken after first flowing the magnetic beads 102 through the reaction chamber 202 followed by flowing *Streptococcus mutans* at a flow rate of 250 μl/min for one second through the reaction chamber 202. FIG. 10C is a photograph taken after flowing the magnetic beads 102 through the reaction chamber 202 followed by flowing *Streptococcus mutans* at a flow rate of 250 μl/min for 60 seconds through the reaction chamber 202.

Referring to FIG. 10A, magnetic beads 102 were captured in the electrode portion of the reaction chamber 202 by DEP. Referring to FIG. 10B, subsequently flowing bacteria at a flow rate of 250 μl/min for one second released some of the magnetic beads 102 previously captured in the reaction chamber 202. Moreover, referring to FIG. 10C, after flowing bacteria at a flow rate of 250 μl/min for 60 seconds, most of the magnetic beads 102 previously captured were released and washed out of the reaction chamber 202 due to the speed of fluid flow.

From this result, it can be seen that capturing cells 502 after capturing magnetic beads 102 may not be desirable because the magnetic beads 102 originally captured were released due to the high speed of the fluid.

After flowing a solution of *Streptococcus mutans* having a density of $10^7$ cells/ml at a flow rate of 250 μl/min for four minutes, magnetic beads in a solution having a density of $10^5$ beads/μl were conveyed at a flow rate of 50 μl/min for one minute, that is, the order of the introduction of the magnetic bead 102 and the cells 502 was reversed compared to the experiment described above.

FIG. 11A is a photograph taken after flowing *Streptococcus mutans* at a flow rate of 250 μl/min for four minutes through a reaction chamber 202. FIG. 11B is a photograph taken after flowing the *Streptococcus mutans* solution through the reaction chamber 202 followed by flowing magnetic beads 102 at a flow rate of 50 μl /min for one minute through the reaction chamber 202. Referring to FIG. 11A, the bacteria 502 were effectively captured in the electrode portion by DEP. Referring to FIG. 11B, magnetic beads 102 were, in addition to the captured cells, evenly and effectively captured between electrodes.

From the result, it can be seen that capturing magnetic beads 102 subsequent to capturing cells 502 is an effective way for capturing both magnetic beads 102 and cells 502.

<Example 6>

Confirmation of cell concentration effect and cytolysis effect of microfluidic device subject to an embodiment of present invention

A PCR was performed in order to measure the quantity of DNA of a sample obtained by using the microfluidic device of the present invention, which has a reaction chamber of 4 μl. Samples were obtained according to the following methods: a) performing cytolysis by laser irradiation after a concentration step using a chip for cell concentration in which a metal electrode is patterned; b) performing cytolysis by laser irradiation without a previous concentration step, using a chip for cell concentration in which a metal electrode is patterned on glass; and c) performing cytolysis by laser irradiation without a previous concentration step, using a cytolysis chip in which a silicon substrate is covered with a glass lid.

When a solution having a density of $10^7$ cells/ml and solution containing magnetic beads are mixed at ratio of 9:1 and put in the 4 μl reaction chamber 202, 36,000 cells are present in the reaction chamber 202. When a voltage is applied to the electrodes of the chamber and further mixture is pumped through the reaction chamber 202, the cells are concentrated in the reaction chamber 202. $10^6$ cells (concentration ratio: 28 times) were present in the reaction chamber 202 after the mixture was conveyed at a rate of 100 $\mu$l /min for one minute. 2 X 10$^6$ cells (concentration ratio: 56 times) were present in the reaction chamber 202 after the mixture was conveyed for two minutes, and 4 X 10$^6$ cells (concentration ratio: 111 times) were present in the reaction chamber 202 after the mixture was conveyed for four minutes.

a) In order to confirm DNA quantity when performing both concentration using DEP and nucleic acid extraction using a laser, a solution containing 10$^7$ cells/ml (0.01 OD) of *Streptococcus mutans* was transported at a flow rate of 100 $\mu$l/min while a voltage of 20 V with a frequency of 100 kHz was applied to the electrodes. Thereafter, a solution having 10$^5$ magnetic beads/$\mu$l was conveyed at a flow rate of 50 $\mu$l/min for one minute, and thereafter the voltage was turned off. The mixture was then irradiated with a 0.8 W laser beam 404 for 40 seconds. The magnetic beads 102 were removed from the sample solution after flowing the solution out of the chamber. Then, PCR was performed by diluting 2 $\mu$l of the sample solution released out of the reaction chamber 202 after laser irradiation with water by a factor of 10. PCR was performed with a LightCycler$_{\circledR}$(Roche Diagnostics Corporation, IN, USA) using 20 $\mu$l of a reaction mixture including a 1X FastStart DNA Master SYBR (Roche Diagnostics Corporation, IN, USA), 0.25 mM forward and reverse primers (Genotech, Republic of Korea), 4 mM MgCl$_2$ (Roche Diagnostics Corporation), and D.W. (PCR class, Roche Diagnostics Corporation, IN, USA)

b) The DNA quantity was confirmed when performing only a nucleic acid extraction step using a laser in the same manner as described above, except that no electrodes were present. A chip with a glass lid bound to a silicon chip was used; cells 502 and beads 102 were supplied together; and the experiment was performed using cell concentrations of 10$^7$ cells/ml (0.01 OD), 10$^8$ cells/ml (0.1 OD) and 10$^9$ cells/ml (1 OD).

c) A glass chip comprising a patterned Au electrode was used for cell concentration using DEP. All the experiment factors of the above method were used identically, except the following: an electric field was not applied; beads 102 and cells 502 were supplied simultaneously; and solutions having a density of 10$^7$ cells/ml (0.01 OD), 10$^8$ cells/ml (0.1 OD) and 10$^9$ cells/ml (1 OD) were applied since the Au of the patterned electrodes could be barrier detaining the laser beam.

The results from the above experiments are shown in Table 1 and FIG. 12. As shown in Table 1 and FIG. 12, the amounts of DNA were similar in both cases b) and c). However, case c) resulted in a higher elution efficiency because the DEP chip had a smaller Cp than the transparent silicone/glass bonded chip. Moreover, when cell concentration was performed using a Au electrode with a DEP chip and a cell lysis by laser irradiation was performed, the Cp value was 6 smaller than when only laser elution without cell concentration was performing. Therefore the amount of DNA was almost 111 times greater than expected.

<Table 1 >

|  |  | Average of Cp | Standard deviation of Cp |
|---|---|---|---|
| DEP chip with Au electrode: performing laser elution after concentration (a) (triangles in FIG. 12) | (0.01 OD) | 14.82 | 0.07 |
| Chip used for laser elution (Si/glass bonded chip) (b) (squares in FIG. 12) | 1 OD | 16.73 | 0.16 |
|  | 0.1 OD | 19.53 | 0.05 |
|  | 0.01 OD | 21.55 | 0.18 |
| DEP chip with Au electrode: performing only laser elution without concentration (c) (circles in FIG. 12) | 1 OD | 16.01 | 0.02 |
|  | 0.1 OD | 18.14 | 0.14 |
|  | 0.01 OD | 21.32 | 0.31 |

As described above, when using the system and the method of the present invention, cells or viruses can be effectively concentrated and lysed. Furthermore, the concentration and lysis of cells or viruses can be performed in one chamber. Therefore, the size of an LOC can be reduced using the microfluidic device and the method according to the present invention.

**Claims**

1. A system for the concentration and the lysis of cells (502) or viruses comprising:

a microfluidic device (100),

said microfluidic device (100) comprising

a reaction chamber (202) suitable for accommodating magnetic beads 102, said reaction chamber (202) comprising a plurality of electrodes (20, 20') which are suitable fo generating a non-uniform electric field in the reaction chamber (202); **characterised in that** said system further comprises a laser source (402), whereby the laser source (402) is arranged to enable radiation of a laser beam (404) onto any magnetic beads (102) which are accommodated in the chamber.

2.  The system of claim 1, further comprising magnetic beads (102).

3.  The system of claim 2, wherein the size of the magnetic beads (102) is 50 nm-1,000 $\mu$m.

4.  The system of claim 2, wherein the size of the magnetic beads (102) is 1-50 $\mu$m.

5.  The system of any of claims 2 to 4, wherein the magnetic beads (102) are a mixture of magnetic beads (102) of two or more different sizes.

6.  The system of any of claims 2 to 5, wherein the magnetic beads (102) comprise one or more materials selected from the group consisting of Fe, Ni and Cr, and oxides thereof.

7.  The system of any of claims 2 to 6, wherein the magnetic beads (102) comprise ferromagnetic metals coated on a polymer, an organic material, silicon, or glass.

8.  The system according to any of claims 2 to 7, wherein the magnetic beads (102) comprise a functional group that is hydrophilic and negatively charged on the surface.

9.  The system of claim 8, wherein the functional group on the surface of the magnetic beads (102) is a carboxy radical or a derivative thereof.

10. The system of claim 1, further comprising two metal pads (10, 10') and a plurality of metal lines (12, 12'), wherein the plurality of electrodes (20, 20') are arranged in at least two rows (11, 11'), each row (11, 11') comprising at least two electrodes (20 and/or 20'), wherein each row (11, 11') is arranged perpendicular to the direction (208) of a fluid flowing through the reaction chamber (202), and wherein the electrodes (20') of the odd rows (11') are connected to the first metal pad (10') via metal lines (12') and the electrodes (20) of the even rows (11) are connected to the second metal pad (10) via other metal lines (12).

11. The system of claim 10, wherein the electrodes (20') of a rows (11, 11') extend into the adjacent row (11, 11') of electrodes (20).

12. The system of claim 10, wherein the electrodes (20') in each of the odd rows (11') and the electrodes (20) in each of the even rows (11) are connected to the first or second metal pad (10', 10), respectively, via the same metal line (12', 12).

13. The system of claim 10, wherein the distance between one of the electrodes (20') of one of the odd rows (11') and the neighboring electrodes (20) of the adjacent even row (11) is 10-100 $\mu$m, the distance between adjacent electrodes (20') within an odd row (11') and an even row (11) is 10-100 $\mu$m, and the height of the electrodes is 0.1-100 $\mu$m.

14. The system of claim 10, wherein the electrodes (20, 20') are columnar.

15. The system of claim 10, wherein the electrodes (20, 20') are flat.

16. The system of claim 10, wherein the electrodes (20, 20') are composed of gold coated on a metal.

17. The system) of claim 16, wherein the metal is nickel.

18. The system of claim 10, wherein the reaction chamber (202) comprises a lower substrate (16) and an upper substrate (18), and the electrodes (20, 20') are disposed on the lower substrate (16) and/or on the upper substrate (18).

19. The system) of claim 18, wherein a surface of the electrodes (20, 20') is arranged at an angle from 50 to 120° to the surface of the substrate (16, 18).

20. The system of claim 18, wherein rows (11, 11') of the electrodes (20, 20') are disposed on the lower substrate (16) and rows (11, 11') of the electrodes (20, 20') are disposed on the upper substrate (18), which rows (20, 20') disposed on the lower substrate and the upper substrate correspond to each other, and wherein the electrodes (20') of odd rows (11') disposed on the lower substrate (16) and the electrodes (20) of the even rows (11) disposed on the upper substrate (18) are connected to the same metal pad (10, 10'), whereas the electrodes (20) of even rows (11) disposed on the lower substrate (16) and the electrodes (20') of odd rows (11') disposed on the upper substrate (18) are connected to the other metal pad (10', 10).

21. The system of claim 1, further comprising a vibrating part (302) for agitating the magnetic beads (102) in the reaction chamber (202).

22. The system of claim 21, wherein the vibrating part (302) is selected from the group consisting of:

   an ultrasonic cleaner;
   a vibrating part using a magnetic field;
   a vibrating part using an electric field;
   a mechanical vibrating unit; and
   a piezoelectric material.

23. The system of claim 1, wherein the laser source (402) is a pulse laser or a continuous wave laser.

24. The system of claim 22, wherein the output power of the pulse laser is more than 1 mJ/pulse and the output power of the continuous wave laser is more than 10 mW.

25. The system of claim 22, wherein the laser (402) generates light in a wavelength band of 400 nm or greater.

26. A method of concentrating and lysing cells (502) or viruses using the microfluidic device (100) according to any of claims 1 to 25, the method comprising:

   a first step of applying a voltage to the electrodes (20, 20') thereby generating a non-uniform electric field in the reaction chamber (202);
   a second step of flowing a fluid containing cells (502) or viruses through the reaction chamber (202) while the voltage of step a) is applied;
   a third step of flowing a fluid containing magnetic beads (102) through the reaction chamber (202) while the voltage of step a) is applied; and
   a forth step of irradiating a laser beam (404) onto the magnetic beads (102) in the reaction chamber (202).

27. The method of claim 26, wherein the applied voltage has a magnitude of 1-100 V and a frequency of 100 Hz-100 MHz.

28. The method of claim 26, wherein flow rate of the fluids flowing through the reaction chamber (202) is 0.1 mm/sec or greater.

29. The method of claim 26, wherein the fluid including the cells (502) or viruses is selected from a group consisting of spittle, urine, blood, blood serum and cell broth.

**Patentansprüche**

1. System zur Anreicherung und zur Lyse von Zellen (502) oder Viren, das umfasst:

   eine mikrofluidische Vorrichtung (100),

   wobei die mikrofluidische Vorrichtung (100) eine Reaktionskammer (202) umfasst, die zur Aufnahme magnetischer Kügelchen (102) geeignet ist, wobei die Reaktionskammer (202) eine Vielzahl von Elektroden (20, 20') umfasst, die zur Erzeugung eines inhomogenen elektrischen Feldes in der Reaktionskammer (202) geeignet sind, **dadurch gekennzeichnet, dass** das System ferner eine Laserquelle (402) umfasst,

wobei die Laserquelle (402) so angeordnet ist, dass eine Bestrahlung mit einem Laserstrahl (404) auf die magnetischen Kügelchen (102), die in der Kammer untergebracht sind, möglich ist.

2. System nach Anspruch 1, das ferner magnetische Kügelchen (102) umfasst.

3. System nach Anspruch 2, wobei die Größe der magnetischen Kügelchen (102) 50 nm-1000 $\mu$m ist.

4. System nach Anspruch 2, wobei die Größe der magnetischen Kügelchen (102) 1-50 $\mu$m ist.

5. System nach einem der Ansprüche 2 bis 4, wobei die magnetischen Kügelchen (102) ein Gemisch aus magnetischen Kügelchen (102) von zwei oder mehr verschiedenen Größen sind.

6. System nach einem der Ansprüche 2 bis 5, wobei die magnetischen Kügelchen (102) ein oder mehr Materialien umfassen, die ausgewählt sind aus der Gruppe bestehend aus Fe, Ni und Cr sowie Oxiden dieser Elemente.

7. System nach einem der Ansprüche 2 bis 6, wobei die magnetischen Kügelchen (102) ein Polymer, ein organisches Material, Silikon oder Glas umfassen, das mit ferromagnetischen Metallen beschichtet ist.

8. System nach einem der Ansprüche 2 bis 7, wobei die magnetischen Kügelchen (102) eine funktionelle Gruppe, die hydrophil und negativ geladen ist, auf der Oberfläche umfassen.

9. System nach Anspruch 8, wobei die funktionelle Gruppe auf der Oberfläche der magnetischen Kügelchen (102) ein Carboxyradikal oder ein Derivat davon ist.

10. System nach Anspruch 1, das ferner umfasst: zwei metallische Anschlussflächen (10, 10') und eine Vielzahl von Metallleitungen (12, 12'), wobei die Vielzahl der Elektroden (20, 20') in wenigstens zwei Reihen (11, 11') angeordnet sind, von denen jede Reihe (11, 11') wenigstens zwei Elektroden (20 und/oder 20') umfasst, wobei jede Reihe (11, 11') senkrecht zu der Richtung (208), entlang der ein Fluid durch die Reaktionskammer (202) fließt, angeordnet ist, und wobei die Elektroden (20') von den ungeraden Reihen (11') mit der ersten metallischen Anschlussfläche (10') über Metallleitungen (12') verbunden sind und die Elektroden (20) von den geraden Reihen (11) mit der zweiten metallischen Anschlussfläche (10) über andere Metallleitungen (12) verbunden sind.

11. System nach Anspruch 10, wobei die Elektroden (20') von einer Reihe (11, 11') sich bis in die benachbarte Reihe (11, 11') von Elektroden (20) hinein erstrecken.

12. System nach Anspruch 10, wobei die Elektroden (20') in jeder der ungeraden Reihen (11') und die Elektroden (20) in jeder der geraden Reihen (11) mit der ersten bzw. der zweiten metallischen Anschlussfläche (10', 10) über die gleiche Metallleitung (12', 12) verbunden sind.

13. System nach Anspruch 10, wobei der Abstand zwischen einer von den Elektroden (20') von einer der ungeraden Reihen (11') und der benachbarten Elektrode (20) von der nächsten geraden Reihe (11) 10-100 $\mu$m ist, der Abstand zwischen benachbarten Elektroden (20') innerhalb einer ungeraden Reihe (11') und innerhalb einer geraden Reihe (11) 10-100 $\mu$m ist, und die Höhe der Elektroden 0,1-100 $\mu$m ist.

14. System nach Anspruch 10, wobei die Elektroden (20, 20') säulenförmig sind.

15. System nach Anspruch 10, wobei die Elektroden (20, 20') flach sind.

16. System nach Anspruch 10, wobei die Elektroden (20, 20') aus einem Metall bestehen, das mit Gold beschichtet ist.

17. System nach Anspruch 16, wobei das Metall Nickel ist.

18. System nach Anspruch 10, wobei die Reaktionskammer (202) einen unteren Träger (16) und einen oberen Träger (18) umfasst, und die Elektroden (20, 20') auf dem unteren Träger (16) und/oder auf dem oberen Träger (18) angeordnet sind.

19. System nach Anspruch 18, wobei eine Oberfläche von den Elektroden (20, 20') in einem Winkel von 50 bis 120° zu der Oberfläche des Trägers (16, 18) ausgerichtet ist.

**20.** System nach Anspruch 18, wobei Reihen (11, 11') von Elektroden (20, 20') auf dem oberen Träger (16) angeordnet sind und Reihen (11, 11') von Elektroden (20, 20') auf dem unteren Träger (18) angeordnet sind, wobei die Reihen (20, 20'), die auf dem unteren Träger und dem oberen Träger angeordnet sind, einander entsprechend angeordnet sind, und wobei die Elektroden (20') von ungeraden Reihen (11'), die auf dem unteren Träger (16) angeordnet sind, und die Elektroden (20) von den geraden Reihen (11), die auf dem oberen Träger (18) angeordnet sind, mit der gleichen metallischen Anschlussfläche (10, 10') verbunden sind, während die Elektroden (20) von geraden Reihen (11), die auf dem unteren Träger (16) angeordnet sind, und die Elektroden (20') von ungeraden Reihen (11'), die auf dem oberen Träger (18) angeordnet sind, mit der anderen metallischen Anschlussfläche (10', 10) verbunden sind.

**21.** System nach Anspruch 1, das ferner eine Vibrationsvorrichtung (302) zum Bewegen der magnetischen Kügelchen (102) in der Reaktionskammer (202) umfasst.

**22.** System nach Anspruch 21, wobei die Vibrationsvorrichtung (302) ausgewählt ist aus der Gruppe bestehend aus:

    einem Ultraschallreiniger;
    einer Vibrationsvorrichtung, die ein Magnetfeld verwendet;
    einer Vibrationsvorrichtung, die ein elektrisches Feld verwendet;
    einer mechanischen Vibrationsvorrichtung; und
    einem piezoelektrischen Material.

**23.** System nach Anspruch 1, wobei die Laserquelle (402) ein gepulster Laser oder ein kontinuierlicher Laser ist.

**24.** System nach Anspruch 22, wobei die Ausgangsleistung des gepulsten Lasers größer als 1 mJ/Puls ist und die Ausgangsleistung des kontinuierlichen Lasers größer als 10 mW ist.

**25.** System nach Anspruch 22, wobei der Laser (402) Licht in einem Wellenlängenband von 400 nm oder größer erzeugt.

**26.** Verfahren zum Anreichern und Lysieren von Zellen (502) oder Viren unter Verwenden der mikrofluidischen Vorrichtung (100) gemäß einem der Ansprüche 1 bis 25, wobei das Verfahren umfasst:

    einen ersten Schritt des Anlegens einer Spannung an die Elektroden (20, 20'), wodurch ein inhomogenes elektrisches Feld in der Reaktionskammer (202) erzeugt wird;
    einen zweiten Schritt des Strömens eines Fluides, das Zellen (502) oder Viren enthält, durch die Reaktionskammer (202), während die Spannung des Schrittes a) angelegt ist;
    ein dritter Schritt des Strömens eines Fluides, das magnetische Kügelchen (102) enthält, durch die Reaktionskammer (202), während die Spannung des Schrittes a) angelegt ist; und
    ein vierter Schritt des Bestrahlens eines Laserstrahles (404) auf die magnetischen Kügelchen (102) in der Reaktionskammer (202).

**27.** Verfahren nach Anspruch 26, wobei die angelegte Spannung eine Größe von 1-100 V und eine Frequenz von 100 Hz-100 MHz hat.

**28.** Verfahren nach Anspruch 26, wobei die Durchflussrate von den Fluiden, die durch die Reaktionskammer (202) fließen, 0.1 mm/s oder größer ist.

**29.** Verfahren nach Anspruch 26, wobei das Fluid, das die Zellen (502) oder Viren enthält, ausgewählt ist aus der Gruppe bestehend aus Speichel, Urin, Blut, Blutserum und Zellkultur.

## Revendications

**1.** Système pour la concentration et la lyse de cellules (502) ou de virus, comprenant:

    un dispositif microfluidique (100),
    ledit dispositif microfluidique (100) comprenant
    une chambre de réaction (202) appropriée pour recevoir des billes magnétiques (102), ladite chambre de réaction (202) comprenant une pluralité d'électrodes (20, 20') qui sont appropriées pour générer un champ électrique non uniforme dans la chambre de réaction (202);

**caractérisé en ce que** ledit système comprend en outre une source de faisceau laser (402), moyennant quoi
la source de faisceau laser (402) est disposée de manière à permettre l'irradiation, par un faisceau laser (404), de billes magnétiques (102) reçues dans la chambre.

2. Système de la revendication 1, comprenant en outre des billes magnétiques (102).

3. Système de la revendication 2, dans lequel la taille des billes magnétiques (102) est de 50 nm à 1000 $\mu$m.

4. Système de la revendication 2, dans lequel la taille des billes magnétiques (102) est de 1 à 50 $\mu$m.

5. Système de l'une quelconque des revendications 2 à 4, dans lequel les billes magnétiques (102) sont un mélange de billes magnétiques (102) ayant deux ou plusieurs tailles différentes.

6. Système de l'une quelconque des revendications 2 à 5, dans lequel les billes magnétiques (102) comprennent un ou plusieurs matériaux choisis dans le groupe constitué de Fe, Ni, et Cr, et des oxydes de ceux-ci.

7. Système de l'une quelconque des revendications 2 à 6, dans lequel les billes magnétiques (102) comprennent des métaux ferromagnétiques appliqués sous forme de revêtement sur un polymère, un matériau organique, du silicium, ou du verre.

8. Système de l'une quelconque des revendications 2 à 7, dans lequel les billes magnétiques (102) comprennent un groupe fonctionnel qui est hydrophile et chargé négativement sur la surface.

9. Système de la revendication 8, dans lequel le groupe fonctionnel sur la surface des billes magnétiques (102) est un radical carboxy ou un de ses dérivés.

10. Système de la revendication 1, comprenant en outre deux plaquettes métalliques (10, 10') et une pluralité de lignes métalliques (12, 12'), où la pluralité d'électrodes (20, 20') sont disposées au minimum en deux rangées (11, 11'), chaque rangée (11, 11') comprenant au moins deux électrodes (20 et/ou 20'), où chaque rangée (11, 11') est disposée perpendiculairement à la direction (208) d'un fluide circulant à travers la chambre de réaction (202), et où les électrodes (20') des rangées impaires (11') sont connectées à la première plaquette métallique (10') via des lignes métalliques (12') et les électrodes (20) des rangées paires (11) sont connectées à la seconde plaquette métallique (10) via d'autres lignes métalliques (12).

11. Système de la revendication 10, dans lequel les électrodes (20') d'une rangée (11, 11') se prolongent jusque dans la rangée (11, 11') adjacente d'électrodes (20).

12. Système de la revendication 10, dans lequel les électrodes (20') de chacune des rangées impaires (11') et les électrodes (20) de chacune des rangées paires (11) sont connectées respectivement à la première ou à la seconde plaquette métallique (10', 10), via la même ligne métallique (12', 12).

13. Système de la revendication 10, dans lequel la distance entre une des électrodes (20') d'une des rangées impaires (11') et les électrodes voisines (20) de la rangée paire (11) adjacente est de 10 à 100 $\mu$m, la distance entre des électrodes (20') adjacentes dans une même rangée impaire (11'), et entre des électrodes (20) adjacentes dans une même rangée paire (11), est de 10 à 100 $\mu$m, et la hauteur des électrodes est de 0,1 à 100 $\mu$m.

14. Système de la revendication 10, dans lequel les électrodes (20, 20') sont colonnaires.

15. Système de la revendication 10, dans lequel les électrodes (20, 20') sont plates.

16. Système de la revendication 10, dans lequel les électrodes (20, 20') sont composées d'or appliqué sous forme de revêtement sur un métal.

17. Système de la revendication 16, dans lequel le métal est le nickel.

18. Système de la revendication 10, dans lequel la chambre de réaction (202) comprend un substrat inférieur (16) et un substrat supérieur (18), et les électrodes (20, 20') sont disposées sur le substrat inférieur (16) et/ou sur le substrat supérieur (18).

**19.** Système de la revendication 18, dans lequel une surface des électrodes (20, 20') fait un angle de 50 à 120° avec la surface du substrat (16, 18).

**20.** Système de la revendication 18, où des rangées (11, 11') des électrodes (20, 20') sont disposées sur le substrat inférieur (16) et des rangées (11, 11') des électrodes (20, 20') sont disposées sur le substrat supérieur (18), lesdites rangées (20, 20') disposées sur le substrat inférieur et le substrat supérieur correspondant les unes aux autres, et où les électrodes (20') des rangées impaires (11') disposées sur le substrat inférieur (16) et les électrodes (20) des rangées paires (11) disposées sur le substrat supérieur (18) sont connectées à la même plaquette métallique (10, 10'), alors que les électrodes (20) des rangées paires (11) disposées sur le substrat inférieur (16) et les électrodes (20') des rangées impaires (11') disposées sur le substrat supérieur (18) sont connectées à l'autre plaquette métallique (10', 10).

**21.** Système de la revendication 1, comprenant en outre un élément vibrant (302) permettant d'agiter les billes magnétiques (102) dans la chambre de réaction (202).

**22.** Système de la revendication 21, dans lequel l'élément vibrant (302) est choisi dans le groupe constitué de:

un nettoyeur à ultrasons;
un élément vibrant utilisant un champ magnétique;
un élément vibrant utilisant un champ électrique;
un vibreur mécanique; et
un matériau piézoélectrique.

**23.** Système de la revendication 1, dans lequel la source de faisceau laser (402) est un laser à impulsions ou un laser à onde continue.

**24.** Système de la revendication 22, dans lequel l'énergie de sortie du laser à impulsions est supérieure à 1 mJ/impuision et la puissance de sortie du laser à onde continue est supérieure à 10 mW.

**25.** Système de la revendication 22, dans lequel le laser (402) génère une lumière dans une bande de longueurs d'onde de 400 nm ou plus.

**26.** Procédé de concentration et de lyse de cellules (502) ou de virus à l'aide du dispositif microfluidique (100) de l'une quelconque des revendications 1 à 25, ledit procédé comprenant:

une première étape consistant à appliquer une tension sur les électrodes (20, 20'), pour générer ainsi un champ électrique non uniforme dans la chambre de réaction (202);
une deuxième étape consistant à faire circuler un fluide contenant des cellules (502) ou des virus à travers la chambre de réaction (202) pendant que la tension de l'étape a) est appliquée;
une troisième étape consistant à faire circuler un fluide contenant des billes magnétiques (102) à travers la chambre de réaction (202) pendant que la tension de l'étape a) est appliquée; et
une quatrième étape consistant à irradier, avec un faisceau laser (404), les billes magnétiques (102) dans la chambre de réaction (202).

**27.** Procédé de la revendication 26, dans lequel la tension appliquée a une amplitude de 1-100 V et une fréquence de 100 Hz-100 MHz.

**28.** Procédé de la revendication 26, dans lequel le débit des fluides circulant à travers la chambre de réaction (202) est de 0,1 mm/s ou plus.

**29.** Procédé de la revendication 26, dans lequel le fluide renfermant les cellules (502) ou des virus est choisi dans le groupe constitué de la salive, de l'urine, du sang, du sérum sanguin et d'un bouillon de cellules.

# FIG. 1

# FIG. 2A

# FIG. 2B

## FIG. 2C

## FIG. 2D

18

# FIG. 3A

# FIG. 3B

PUMP

POWER
SUPPLY

# FIG. 4

5μm W D

# FIG. 5A

(W,P):(25,25)

# FIG. 5B

(W,P):(25,9)

# FIG. 5C

(W,P):(5,25)

# FIG. 5D

(W,P):(12,25)

# FIG. 6

# FIG. 7

# FIG. 8A

# FIG. 8B

# FIG. 9

FLOW RATE (ul/min)

CONCENTRATION RATIO

FLOW VELOCITY (mm/sec)

FIG. 10A

FIG. 10B

FIG. 10C

## FIG. 11A

Trapped S.M.

## FIG. 11B

Trapped magnetic beads

# FIG. 12

CELL DENSITY (cell/volume of chamber)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004018611 A
- WO 0212896 A